Europäisches Patentamt
European Patent Office
Office européen des brevets

(19) (11) Publication number: **0 227 909 A1**

(12) **EUROPEAN PATENT APPLICATION**

(21) Application number: **86114978.9**

(51) Int. Cl.⁴: **C12N 15/00**

(22) Date of filing: **28.10.86**

(30) Priority: **28.10.85 US 791905**
**16.10.86 US 919762**

(43) Date of publication of application:
**08.07.87 Bulletin 87/28**

(84) Designated Contracting States:
**AT BE CH DE ES FR GB GR IT LI LU NL SE**

(71) Applicant: **THE GENERAL HOSPITAL CORPORATION**
**55 Fruit Street**
**Boston MA 02114(US)**

(72) Inventor: **Goodman, Howard M.**
**10 The Ledges Road**
**Newton Center Massachusetts 02159(US)**
Inventor: **Cheng, Chi-Lien**
**2 Ware Street, No. 401**
**Cambridge Massachusetts 02138(US)**

(74) Representative: **Patentanwälte Grünecker, Kinkeldey, Stockmair & Partner**
**Maximilianstrasse 58**
**D-8000 München 22(DE)**

(54) **Polynucleotides coding for nitrate reductase.**

(57) The present invention provides DNA molecules and fragments coding for nitrate reductase. The invention provides DNA fragments comprising a non-functional part of the cDNA sequence (fragment type A), a functional cDNA sequence (fragment type B), or a functional chromosomal gene (fragment type C) of nitrate reductase. The invention also provides for vehicles capable of replication and expression comprising the type A, B, or cDNA molecules and fragments coding for nitrate reductase. The invention further provides for hosts transformed with said vehicle and with said type A, B, or cDNA molecules and fragments coding for nitrate reductase.

In another embodiment, the invention provides for plants and plant cells and methods thereof, such that the level of nitrate reductase activity of said plants and plant cells is increased.

NITRATE REDUCTASE
IS INDUCED BY NITRATE

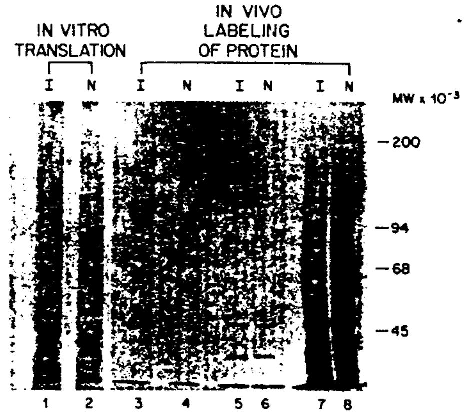


FIG. 1

EP 0 227 909 A1

Xerox Copy Centre

# POLYNUCLEOTIDES CODING FOR NITRATE REDUCTASE

## BACKGROUND OF THE INVENTION

This application is a continuation-in-part of United States Patent Application Serial No. 791,905 filed October 28, 1985, the contents of which are fully incorporated herein by reference.

### Field of the Invention

The present invention relates to the field of recombinant DNA technology and to its uses in the isolation, cloning, and expression of the cDNA or of chromosomal genes coding for nitrate reductase.

### Background of the Invention

In the synthesis of amino acids, different organisms utilize and require different forms of nitrogen. Many vertebrates utilize ammonia as a nitrogen source for the synthesis of the nonessential amino acids, but are unable to use nitrite, nitrate, or $N_2$. Higher plants can synthesize amino acids required for protein synthesis, using either ammonia or nitrate as a nitrogen source. Much of the nitrate used by these plants is derived from the soil after oxidation of nitrogen to nitrate by soil bacteria. Leguminous plants, on the other hand, harbor symbiotic bacteria in their root nodules and are able to fix molecular nitrogen from the atmosphere as ammonia, which is then used for amino acid synthesis.

Microorganisms vary considerably in their capacity to synthesize amino acids. Most microorganisms, however, require a reduced form of nitrogen, such as ammonia, but numerous bacteria and fungi can also utilize nitrite or nitrate.

Nitrate, as a nitrogen source, is first reduced to nitrite and the nitrite is then reduced to ammonia. Nitrate reduction is catalyzed by nitrate reductase (NR), which is ubiquitous in plants and microorganisms. This enzyme is a molybdenum-containing flavoprotein and employs NADPH (nicotinamide adenine dinucleotide phosphate-reduced). The characteristic reaction of nitrate reductase is as follows:

$$\text{NADPH} \longrightarrow \text{FAD} \longrightarrow \text{Mo} \longrightarrow NO_3$$

(FAD is flavin adenine dinucleotide).

The reduction of nitrite to ammonia by nitrite reductase of plants requires a highly electronegative reductant. In green plants, ferredoxin reduced during the light reactions may serve as the ultimate reductant of nitrite in the following chain of reactions:

$$\text{Ferredoxin} \longrightarrow \text{NADP} \rightarrow \text{FAD} \longrightarrow NO_2$$

Free ammonia ($NH_3$) is formed as the end product, which is then utilized to aminate alpha-ketoglutarate and thus to provide amino groups by transamination. A thorough discussion of nitrate reductase is given in L. Beevers and R. Hageman, "Nitrate and Nitrite Reduction," Chapter 3 of The Biochemistry of Plants, Vol. 5 (Academic Press, Inc. 1980) at pages 115 to 168, incorporated herein by reference.

The nitrogen level, and hence the growth and yield of crop plants, correlates with the nitrate reductase level of the plants. Nitrate reductase levels fluctuate in response to a number of environmental factors such as temperature and light. Further, nitrate reductase is the first higher plant enzyme that shows induction by its substrate, nitrate. Filner, P., "Regulation of Nitrate Reductase in Cultured Tobacco Cells," Biochim., Biophys. Acta, 118: 299-310 (1966), showed that nitrate induction involves *de novo* synthesis of nitrate reductase protein. Induction by nitrate also increases the nitrate reductase protein activity as described by

Somers et al., "Synthesis and Degradation of Barley Nitrate Reductase," Plant Physiol., 72: 949-52 (1982). Induction of nitrate reductase is also described in Mendel et al., "Molybdenum Cofactor in Nitrate Reductase--Deficient Tobacco Mutants. III, Induction of Cofactor Synthesis by Nitrate," Plant Science Letters, 27: 95-101 (1982).

Nitrate reductase-deficient mutants can be used as a selection marker in recombinant DNA techniques. Moreover, using recombinant DNA techniques, the level of nitrate reductase activity in plants may be increased.

## SUMMARY OF THE INVENTION

The present invention provides DNA molecules and fragments coding for nitrate reductase. The invention provides DNA fragments comprising a non-functional part of the cDNA sequence (fragment type A), a functional cDNA sequence (fragment type B), or a functional chromosomal gene (fragment type C) of nitrate reductase of barley seedlings (Hordeum vulgare). The invention also provides for vehicles capable of replication and expression comprising the type A, B, or C DNA molecules and fragments coding for nitrate reductase. The invention further provides for hosts transformed with said vehicle and with said type A, B, or C DNA molecules and fragments coding for nitrate reductase.

In another embodiment, the invention provides for plants and plant cells and methods thereof, such that the level of nitrate reductase activity of said plants and plant cells is increased.

## DESCRIPTION OF THE FIGURES

Figure 1 illustrates SDS-PAGE gels of the induction of nitrate reductase by nitrate of labeled protein in vivo followed by immuno-precipitation using nitrate-reductase specific polyclonal antibodies (lanes 3 and 4). mRNA from nitrate-induced and non-induced barley seedlings was translated in vitro and the products immunoprecipitated using nitrate reductase specific polyclonal antibodies (lanes 1 and 2).

Figure 2 illustrates the fractionation of nitrate reductase mRNA on a sucrose gradient. The upper panel shows mRNA translation. The lower panel shows the 110 Kd molecular weight (mw) nitrate reductase protein following immunoprecipitation.

Figure 3 illustrates the fractionation of nitrate reductase mRNA in a methylmercury hydroxide agarose gel. The upper panel shows the mRNA translation. The lower panel shows the 110 Kd molecular weight - (mw) nitrate reductase protein following immunoprecipitation.

Figure 4 shows the northern analysis of mRNA from nitrate-induced and non-induced barley seedlings using cDNA from bNR10 as probe.

Figure 5 shows a SDS-PAGE gel of the 110 Kd molecular weight (mw) nitrate reductase protein after hybrid selection with cDNA from bNRp10 followed by in vitro translation and immunoprecipitation.

Figure 6 shows the restriction mapping of the cDNA clones bNRp10, bNRλ1, and bNRλ2.

## DEFINITIONS

In the description that follows, a number of terms in recombinant DNA technology are extensively utilized. In order to provide a clear and consistent understanding of the specification and claims, including the scope to be given such terms, the following definitions are provided.

Nucleotide. A monomeric unit of DNA or RNA consisting of a sugar moiety, a phosphate, and a nitrogenous heterocyclic base. The base is linked to the sugar moiety via the glycocytic carbon (1' carbon of the pentose). The combination of a base and a sugar is called a nucleoside. Each nucleotide is characterized by its base. The four DNA bases are adenine (A), guanine (G), cytosine (C) and thymine (T). The four RNA bases are A, G, C, and uracil (U).

DNA or Genetic Sequence. A linear array of nucleotides connected one to the other via phosphodiester bonds between the 3' and 5' carbons of adjacent pentoses.

Functional Genetic Sequence. A genetic sequence coding for a polypeptide having desired activity, regardless of whether the sequence is shorter or longer than that of the full length sequence for the polypeptide. It is also referred to as a "functional gene."

Codon or Triplet. A sequence of three nucleotides which encodes an amino acid, a translation start signal or a translation termination signal. For example, messenger codons TTA, TTG, CTT, CTC, CTA, and CTG encode for the amino acid leucine. TAG, TAA, and TGA are translation stop signals. ATG is a translation start signal..

Reading Frame. The grouping of codons during translation of mRNA into amino acid sequence. During translation, the proper reading frame must be maintained. For example, the sequence GCTGGTTGTAAG may be translated into three reading frames or phases depending on whether one starts with G, with C or with T, and thus may yield three different peptide products.

Structural Gene. A DNA sequence which encodes through its template or mRNA a sequence of amino acids characteristic of a specific peptide.

Transcription . The process of producing mRNA from a structural gene.

Translation. The process of producing a polypeptide from mRNA.

Expression. Expression is the process by which a structural gene produces a polypeptide. It involves transcription of the gene into mRNA and the translation of such mRNA into the polypeptide.

Cloning Vehicle. A plasmid or phage DNA or other DNA sequences which are able to replicate in a host cell, which are characterized by one or a small number of endonuclease recognition sites at which such DNA sequences may be cut in a determinable fashion without loss of an essential biological function of the DNA, and which contain a phenotypic selection marker suitable for use in the identification of transformed cells. Markers, for example, are tetracycline resistance or ampicillin resistance. Phenotypic markers can also include for the purposes of the present invention conferring nitrate reductase activity to nitrate reductase-deficient mutant host cells. The word "vector" is sometimes used for a cloning vehicle.

Expression Vehicle. A vehicle similar to a cloning vehicle, but which is capable of expressing a given structural gene in a host, normally under control of certain regulatory sequences.

Replication Vehicle. A cloning or expression vehicle.

Plasmid. A non-chromosomal double-stranded DNA sequence comprising an intact "replicon" such that the plasmid is replicated in a host cell. When the plasmid is placed with a unicellular organism, the characteristics of that organism may be changed or transformed as a result of the DNA of the plasmid. For example, a plasmid carrying the gene for tetracycline resistance transforms a cell previously sensitive to tetracycline into one which is resistant to it.

Expression Control Sequence. A sequence of nucleotides that controls or regulates expression of structural genes when operably linked to those genes. They include the lac system, the trp system, major operator and promoter regions of phage lambda, the control region of fd protein, and other sequences known to control the expression of genes and procaryotic or eucaryotic cells.

Plant Promoter. An expression control sequence which is capable of causing the expression in said plant of any homologous or heterologous genetic sequence or sequences operably linked to such promoter.

Cloning. The process of obtaining a population of organisms or DNA sequences derived from one such organism or sequence by asexual reproduction.

Host. Any organism that is the recipient of a replicable expression vehicle, including procaryotes and eucaryotes.

Heterologous Gene. A gene that is foreign, i.e. originating from a donor different from the host or a chemically synthesized gene and can include a donor of a different species from the host. The gene codes for polypeptides ordinarily not produced by the organism suseptible to transformation by the expression vehicle.

Nitrate Reductase. The definition of this enzyme is functional, and includes any nitrate reductase capable of functioning as a selectable marker by conferring nitrate reductase activity to a nitrate reductase-deficient host cell. The definition also includes any nitrate reductase capable of functioning in a given desired plant to increase the nitrate reduc tase activity of said plant. The term therefore includes not only the specific enzyme from the specific plant species involved in the genetic transformation, but may include nitrate reductase from other plant species or microbes or even other eucaryotes, if such nitrate reductase is capable of functioning in the transformed plant cells.

DESCRIPTION OF THE PREFERRED EMBODIMENTS

The present invention is based on the discovery of cDNA fragments encoding nitrate reductase (NR). These cDNA fragments for nitrate reductase constitute the basis for the preparation of the functional nitrate reductase gene. The cDNA fragments of the present invention were isolated from two separate libraries.

In one library enriched mRNA for nitrate reductase was used to construct a cDNA library in the λgt11 vector (Young, R.A. and Davis, R.W. PNAS , 80 :1194-1198 (1983). The recombinants were screened for the expression of antigenic sites which could be recognized by the antibody against nitrate reductase. Two independent recombinant clones showed cross-reactivity to nitrate reductase antibody. The cDNA of these two clones also cross-hybridized to each other. The two recombinant clones from the λgt11 vector were named "bNRλ1" and bNRλ2" (i.e. bNRλ1 vector and bNRλ2 vector).

In the second library, a highly enriched nitrate reductase mRNA was used in constructing a cDNA library in the plasmid vector pUC12 (Vieira, J. and Messing, J., Gene, 19:259-268 1982). The largest recombinant clone obtained from this library contained a 1.1 Kb cDNA insert when screened with bNRλ1 and bNRλ2 probes. This recombinant clone from the pUC12 vector was named "bNRp10 (i.e., bNRp10 vector).

The NR cDNA fragments (hereinafter "NR fragment") present in the vector λgt11 and in plasmid vector pUC12, as will be obvious to one of skill in genetics, are not the only form which these fragments can take. Thus, genetic information of type A, B, or C can, using readily available techniques, be prepared by synthetic methods.

The NR fragment may, in addition, be present by itself, or in the particular plasmid described above, or in other replication vehicles, or, most importantly, may be part of longer fragments, e.g., type B, containing a functional cDNA, or part of a functional chromosomal gene, e.g., type C, for nitrate reductase.

In turn, a functional cDNA gene (fragment of type B) coding for nitrate reductase, which may comprise the NR fragment may also be in operable linkage with expression control sequences. Thus, combinations of the NR fragement or other fragments of type A with additional polynucleotide sequences in the context of a functional gene, together with regulatory signals for expression, replication, amplification, and regulated response to a variety of conditions and reagents are also included in the present invention.

The DNA fragments which are preferred are those based on cDNA which, by the nature of its origin, does not contain any naturally occurring introns. Another preferred material is messenger RNA which has been matured and is capped, and includes a poly A-3' chain. A third preferred material is the functional chromosomal gene of nitrate reductase.

It is to be understood that the basic material isolated from the cDNA libraries constitutes the starting point for a number of embodiments of the present invention. The NR fragment present therein can, of course, be present in the multiple and various contexts, combinations, and permutations previously described, all of which are covered by the present invention.

The polynucleotide cDNA fragments of type A, including the NR fragment, partially coding for nitrate reductase, find their utilities as probes for the detection, identification, and characterization of the functional cDNA and chromosomal genes coding for nitrate reductase.

The DNA fragments of type B, functional cDNA, and type C, functional chromosomal DNA, which may include the NR fragments of type A, may be used in selectable marker techniques in recombinant DNA methods. For example, the type B or type C fragments, usually in a replication vehicle are used to transform host cells that are deficient in nitrate reductase. The transformed host cells are grown and selected for nitrate reductase production. Subsequently, transformed host cells that show nitrate reductase activity can be induced using nitrate, or repressed using ammonia or nitrite.

Functional cDNA (fragment of type B) and functional chromosomal gene (fragment of type C) can readily be obtained by known methodologies.

The preparation of a functional cDNA clone of type B can take a variety of routes. For example, the NR fragment can be used to screen a cDNA library in lambda gt11 (about 1-3 x $10^6$ clones), or a nitrate reductase cDNA library in pUC12 (about 1-3 x $10^6$ clones). The development of pUC vectors has been described by Vieira, J. and Messing, J., in Gene, 19: 259-268, 1982. A pUC12 library is readily prepared by priming poly (A) RNA from nitrate reductase with oligo dT, reverse transcribing, size selecting on an alkaline sucrose gradient, synthesizing double stranded DNA with DNA polymerase I Klenow fragment, treating with S1 nuclease, tailing with dC, size selecting the DNA on an agarose gel, and introducing the fragments into the dG tailed Pst I site of the vector (pUC12) (Maniatis, T. et al., see page 20 and Chandrda T. et al., see page 20). Up to 1-3 x $10^6$ independent clones are available in such a library. Isolated clones are characterized by mapping and sequencing. Overlapping sequences are determined and newly identified parts which are not covered by the NR fragment are used to rescreen the cDNA libraries. Finally, all different subclones are assembled into a functional cDNA fragment of type B. The subclones or assembled clones are tested for the presence of functional sequences coding for nitrate reductase, by introducing the sequences into appropriate expression systems.

It is conceivable that a functional cDNA clone cannot be obtained by just screening existing cDNA libraries and assembling these clones, since the mRNA coding for nitrate reductase appears to be of consid erable length (3-4 Kb). In that event, methods exist in the art to remedy such a problem. To overcome the very low abundance of specific mRNA, several enrichment procedures can be employed. Nitrate reductase specific mRNA is enriched by hybrid selection, by size selection, by overexpressing a complete genomic copy in an appropriate cell line, or by a combination of these methods. An oligonucleotide (20-30 bp) complementary to nitrate reductase mRNA is synthesized. This oligonucleotide should map close (at a distance of about 20-50 nucleotides) to the 5'-end of the already cloned and identified region. After annealing this primer to RNA enriched for nitrate reductase message, cDNA is obtained by reverse transcription (Maniatis et al., supra ). The cDNA/RNA hybrid is tailed with dC, and the RNA is removed by alkaline hydrolysis or digested with RNase H. Double-stranded cDNA can be cloned into an appropriate vector either after synthetic linker addition or after a second tailing step with dC (Maniatis et al. , supra). A probe for screening for N-terminally coding NR cDNA inserts (type A) can be generated as a synthetic oligonucleotide which is complementary to a region between the previously identified 5'-end and the primer used for reverse transcription. If the 5'-primed region of a functional gene has been identified for a genomic clone, this fragment can also serve as a suitable probe in screening for upstream coding cDNA fragments.

In this manner, a functional clone of the nitrate reductase cDNA and/or a functional chromosomal gene can be identified and isolated, and then introduced into appropriate cloning and/or expression vehicles and hosts in order to express nitrate reductase by microbiological fermentation, in tissue culture or in a whole animal by microinjection of fertilized eggs (Palmiter et al., Science, 222: 809 (1983)). It may further be desirable to tailor fragments of types B and C, containing the functional cDNA and functional chromosomal gene, respectively, for nitrate reductase, by removing non-essential or non-coding portions.

The genetic constructs and the methods involved herein can be utilized for expression of the nitrate reductase in eucaryotic hosts that are deficient in nitrate reductase. Nitrate reductase reduces chlorate to chlorite, a compound toxic to plant cells. Nitrate reductase deficient cells, however, are resistant to chlorate. Thus, mutant nitrate reductase deficient cell lines can be selected as host cells. Morton et al. , "Nitrate Reductase Deficient Cell Lines from Haploid Protoplast Cultures of Nicotiana plumbaginifolia," Mol. Gen. Genet., 182: 301-304 (1982); Muller et al., "Isolation and Characterization of Cell Lines of Nicotiana tabacum Lacking Nitrate Reductase," Mol. Gen. Genet., 161: 67-76 (1978). Examples of nitrate reductase-deficient mutants include Aspergillus nidulans, Neurospora crassa, tobacco mutants, (for example, Nicotiana tabacum), barley mutants, (for example, Hordeum vulgare), and Arabadopsis thaliana.

The nitrate reductase, either functional cDNA or chromosomal DNA, operably linked to a promoter can be recombined with a suitable transfer vehicle, such as tumor-inducing (Ti) plasmid to transfer NR activity to mutant eucaryotic cells. Transformed cells can be selected based on nitrate reductase activity, as shown by cell growth in a culture medium containing nitrate, as the only nitrogen source. The transformed host can be fermented and cultured according to means known in the art to achieve optimal cell growth.

In a preferred embodiment of this invention, the NR fragment or functional cDNA or genomic DNA for NR may be used to increase nitrate reductase activity in grain crops, for example, resulting in grain production of more grain or grain protein. As is known, nitrate reductase activity is correlated with grain and grain protein production, such as in corn, wheat, sorgum, soy beans, and rye grass. Thus, the claimed nitrate reductase can be used to increase the production of grain or grain protein. L. Bevers and R. Hageman, "Nitrate and Nitrite Reduction," supra at 158-159.

One method of introducing genetic material into plant cells is to infect a wounded leaf of the plant with Agrobacteriumtumefaciens bacteria. Under appropriate growth conditions, a ring of calli forms around the wound. The calli are then transferred to growth medium, allowed to form shoots, roots and develop further into plants.

An alternative method to introduce multiple copies of the NR gene into the plant cell is by self-ligating a copy of a functional NR (genomic or cDNA) structural gene, utilizing methods well known to those of skill in recombinant DNA technology. The structural genes, each containing its individual promoter, are operably linked to each other by means of appropriate linkers, and 10-30 copies of each gene can in certain instances be introduced into an appropriate replicable expression vehicle, such as tumor inducing (Ti) plasmid.

Alternatively, the genetic material can be microinjected directly into plant embryo cells or by electroporation as described in Fromm et al., "Expression of Genes Transformed into Monocot and Dicot Plant Cells by Electroporation," Proc. Nat'l. Acad. Sci., USA, 82: 5824-28 (1985) or by direct precipitation using polyethylene glycol as described in Paszkowski et al., EMBO J., 3: 2717-22 (1984).

In the case of monocotyledonous plants, pollen may be transformed with total DNA or an appropriate functional clone providing resistance, and the pollen then utilized to produce progeny by sexual reproduction.

Any other methods utilizable to introduce and increase nitrate reductase activity in a given cell can be utilized. Of particular interest in this invention is the operable linkage of a genetic sequence coding for a structural nitrate reductase gene to another genetic sequence capable of overproduction of the gene product derived therefrom. This linkage of genetic sequences can be introduced into appropriate plant cells, for example, by means of the Ti plasmid.

The introduction of genetic material into plant cells, especially by use of the Ti plasmid of Agrobacterium tumefaciens, is a reproducible and predictable technolgy (See, for example, Caplan, A. et al., Introduction of Genetic Material into Plant Cells," Science, 815-821 (November, 1983); Schell, J. and Van Montagu, M., "The Ti Plasmid as Natural and as Practical Gene Vector for Plants," Bio/Technology, April 1983, pp. 175-180; Horsch et al., "Inheritance of Functional Foreign genes in Plants," Science, 233: 496-498 (1984); Fraley, R. T. et al., Proc. Nat. Acad. Sci. USA, 80: 4803 (1983); Watson et al., Recombinant DNA. A Short Course , Scientific American Books, 1983, pp. 164-1973; and Old and Primrose, Principles of Gene Manipulation, 2d Ed., U. Cal. Press, 1981, pp. 138-156, herein being incorporated by reference).

Ti plasmids contain two regions essential for the production of transformed cells. One of these, named transfer DNA (T DNA), induces tumor formation. The other, termed virulent region, is essential for the formation but not maintenance of tumors. Transfer DNA, which transfers to the plant genome, can be increased in size by the insertion of the functional nitrate reductase gene or of a gene combination without its transferring ability being affected. By removing the tumor-causing genes so that they no longer interfere, the modified Ti plasmid can then be used as a vector for the transfer of the gene constructs of the invention into an appropriate plant cell. The foreign DNA to be inserted is usually introduced between the terminal sequences flanking the T-region.

A particularly useful Ti plasmid vector is pGV3850, a non-oncogenic derivative of the nopaline Ti plasmid C58. (See Caplan et al., supra) This vector utilizes the natural transfer properties of the Ti plasmid. The internal T DNA genes that determines the undifferentiated crown gall phenotype have been de leted and are replaced by any commonly used cloning vehicle (such as pBR322). The cloning vehicle sequence containing between T DNA border regions serves as a region of homology for recombination to reintroduce foreign DNA cloned in a derivative of the same cloning vehicle. Any gene construct of the invention cloned in such plasmid can thus be inserted into pGV3850 by a single recombination of the homologous sequences. Antibiotic resistance markers can be added to the plasmid to select for the recombination event. The presence of the nopaline synthase (nos) gene in this vector makes it easy to monitor the efficiency of transformation using pGV3850. A callus in tissue culture can then be tested for the presence of nopaline.

After transformation of the plant cell or plant, the same may be selected by aid of an appropriate marker, such as antibiotic resistance, or more relevant, increased nitrate reductase activity, and then grown in conventional ways.

Other systems, such as cauliflower mosaic virus, CaMV (Hohn, B. et al., in "Molecular Biology of Plant Tumors," Academic Press, New York, pp. 549-560; and Howell, United States Patent 4,407,956) can also be used. The entire CaMV viral DNA genome is inserted into a parent bacterial plasmid creating a recombinant DNA molecule which can be propagated in bacteria. After cloning, the recombinant plasmid is cleaved with restriction enzymes either at random or at unique sites in the viral portion of the recombinant plasmid for insertion of the gene of the invention. A small oligonucleotide, described as a linker, having a unique restriction site may also be inserted. The modified recombinant plasmid again may be cloned and further modified by introduction of larger pieces of the NR gene or construct thereof into the unique restriction site of the linker. The modified viral portion of the recombinant plasmid is then excised from the parent bacterial plasmid, and used to inoculate the plant cells or plants. This virus is described in the aforementioned Howell patent as being particularly good for insertion of genes capable of enhanced production of protein, greater tolerance to stress, resistance to pests and pesticides, nitrogen fixation, and the like.

Having now generally described the invention, the same will be further illustrated by means of specific examples which are presented herewith for purposes of illustration only and are not intended to be limiting thereof, unless otherwise specified.

## EXAMPLES

### EXAMPLE 1

Induction of the Nitrate Reductase Enzyme By the Nitrate Substrate

In higher plants, nitrate reductase (NR) activity is induced by its substrate, nitrate. Barley seedlings - (Hordeum vulgare) were used to study the regulation of NR, especially its induction by nitrate. Nitrate reductase protein exhibits a single protein band of 110,000 Kd molecular weight on an SDS-polyacrylamide gel electrophoresis. The barley nitrate reductase (native molecular weight of 221,000) therefore consists of two identical subunits. Kuo et al., "NADH-Nitrate Reductase in Barley Leaves, Identification and Amino Acid Composition of Subunit Protein," Biochim., Biophys. Acta, 118: 299-310 (1982).

Barley seedlings were labeled in vivo and induced with nitrate. Nitrate reductase increased markedly upon nitrate induction, and the NR was immunoprecipitated by a nitrate reductase specific polyclonal antibody. On 7% SDS-polyacrylamide gels, as shown in Figure 1, the nitrate reductase 110 Kd band increased in intensity markedly upon nitrate induction. Figure 1, lanes 3-8, shows the in vivolabeling of protein in barley seedlings, followed by immunoprecipitation. In lanes 3 and 4, the nitrate reductase specific polyclonal antibodies were used to immunoprecipitate the protein. Lane 3, induced (I) with nitrate reductase, shows the induced nitrate reductase product, with the band at 110 Kd molecular weight. Lane 4, non-induced (N), does not show the induced nitrate reductase product. In lanes 5 and 6, pre-immune serum was used as a control. Lanes 7 and 8 show the results of the in vivo labeled protein, without immunoprecipitation. Lane 7 shows the induced nitrate reductase product.

To further examine this induction with nitrate, mRNA was extracted from nitrate induced and non-induced barley seedlings. Two populations of mRNA were translated in vitro and their products immunoprecipitated with nitrate reductase specific polyclonal antibodies. The observed level of induction is comparable to that exhibited in vivo in Figure 1. Lanes 1 and 2 of Figure 1, shows the in vitro translation of mRNA. Only the induced mRNA, lane 1, showed the 110 Kd molecular weight nitrate reductase product.

### EXAMPLE 2

Cloning the Nitrate Reductase Gene

To further study the nature of nitrate induction, cloning of the nitrate reductase gene was initiated. One approach taken to cloning the nitrate reductase gene was first to clone the cDNA corresponding to nitrate reductase, and once this cDNA was identified, it could then be used as a probe to isolate the NR gene. As the size of the NR protein was 110 Kd, the estimated minimum size of the mRNA would be 3 Kb. From the in vitro translation and immunoprecipitation experiment, it was learned that the level of NR mRNA in the total mRNA population is low, i.e. about 0.01%. Based on these two pieces of information, it was decided to physically enrich the NR mRNA by taking advantage of its relatively large size. Total mRNA from induced plants was size fractionated on a native sucrose gradient. The location of NR mRNA was determined by in vitro translation of the fractionated mRNA, followed by immunoprecipitation. Figure 2 shows the fractionation of mRNA on the sucrose gradient. The upper panel shows the translation of the purified mRNA. (Every fifth fraction was translated.) The bottom panel of Figure 2 shows the 110 Kd nitrate reductase protein resulting from the immunoprecipitation. A 5-to 10-fold enrichment was achieved by this step. The population of poly-(A+) enriched for NR mRNA was used to construct a cDNA library in the λgt11 vector. 40,000 recombinants were screened with NR antibody for the expression of antigenic sites which could be recognized by the barley antibody against NR. Two independent clones (bNRλ1 and (bNRλ2) showed cross-reactivity to NR antibody. The cDNA of these two clones cross-hybridize to each other, suggesting a common origin.

Simultaneously, another approach was taken to cloning the nitrate reductase gene. The sucrose gradient enriched mRNA population was further enriched for NR by methylmercury hydroxide agarose gel electrophoresis as shown in Figure 3. This method was a separation based on size of the mRNA. The alternative separation methods, one based on sedimentation in a native sucrose gradient as shown in Figure 2, and the other size fractionation by gel electrophoresis as shown in Figure 3, allowed a total enrichment of about 100-fold. (The level of unenriched mRNA was 0.01-0.02% of mRNA poly(A+). Using the sucrose gradient fractionation, the level of mRNA poly(A+) was 0.05-0.1%. The sucrose gradient fractionation plus the methylmercury hydroxide agarose gel fractionation level of mRNA was 1-2%.)

The location of NR mRNA in the gel was again determined by in vitro translation of the mRNA extracted from gel slices. One hundred ng of this highly enriched mRNA was used in constructing a cDNA library in the plasmid vector pUC12 (Vieria and Messing, 1982) using GC tailing. About 4,000 recombinant clones were obtained. This library was initially intended for screening by direct hybrid selection. This library was later screened with bNRλ1 and bNRλ2 from the λgt11 library. Positive clones were obtained from the pUC12 library when 1,000 clones were screened. The number of positive clones in this library - (approximately 1%) confirmed the estimation of the gradient and gel enrichment steps. The largest cone, bNRp10, among these ten clones contained approximately a 1.1 Kb cDNA insert.

EXAMPLE 3

Northern Analysis Using cDNA from bNRp10

Northern analysis using cDNA from bNRp10 as a probe was determined. The size of the mRNA is approximately 3.5 Kb, which is in agreement with the apparent size observed in the methylmercury gel. The results of the Northern analysis are shown in Figure 4, with the probe 2E7 as a control. The mRNA corresponding to bNRp10 is induced by nitrate addition to the plants. These two lines of evidence indicate that bNRp10 is in fact a clone for NR mRNA.

EXAMPLE 4

Hybrid Selection with cDNA bNRp10 Followed by In Vitro Translation and Immunoprecipitation

Immobilized bNRp10 cDNA was able to hybrid select specifically a mRNA which produces a protein of the same size as NR and is immunoprecipitable with NR specific polyclonal antibody. Figure 5 shows the hybrid selection using cDNA bNRp10 followed by in vitro translation and immunoprecipitation. The cDNA clone was immobilized on nitrocellulose paper and used to hybrid select the nitrate reductase mRNA. Lane1 of Figure 5 shows in vitro translation of mRNA with no added messenger (mock translation). Lane 2 is the in vitro translation with hybrid selected messenger, showing the 110 Kd molecular weight nitrate reductase. Lane 3 shows the hybrid selection using the vector along (pUC12) without the insert, immobilized to the filter. Lane 4 shows the immunoprecipitation of the product of lane 2. Lane 5 shows the immunoprecipitation of lane 3. Lane 6 shows the immunoprecipitation of lane 7. Lane 7 is the in vitro translation of the total poly A+RNA (not hybrid selected). Lane 8 is the message after hybrid selection (-NR) using bNRp10 (i.e. the RNA that did not bind to the filter in lane 1).

EXAMPLE 5

Subcloning, Sequencing and Restriction Mapping

Clones bNRλ1 and bNRλ2 were digested with EcoRI and the NR-hybridizing DNA inserts subcloned into the single-stranded vector, M13 mp18 in both orientations. The size of the insert in bNRλ1 is approximately 0.4-0.5 Kb and in bNRλ2 is approximately 0.3-0.4 Kb. DNA sequence was obtained using the universal M13 external primer and the Sanger method of dideoxy sequencing. The approximately 1.1 Kb insert in the pUC12 clone, bNRp10, was digested with SalI and with HindIII (in the polylinker) and SalI and subcloned into appropriately cut M13 mp18. The DNA sequence was then obtained as described above.

The recombinant cloning vectors bNRλ1, bNRλ2 and bNRp10 have been deposited prior to the filing date of this application at the applicant's depository at the Department of Molecular Biology, Massachusetts General Hospital, Boston, Massachusetts and at the American Type Culture Collection (ATCC), Rockville, Maryland, U.S., on October 7, 1986, and given ATCC accession numbers as follows: bNRλ1 is ATCC 40275; bNRλ2 is ATCC 40274; and bNRp10 is ATCC 40276.

Figure 6 shows the restriction mapping of the bNRp10, and the bNRλ1 and bNRλ2.

Sequencing of the bNRλ1 and bNRλ2 clones was then carried out, the results for the combined sequence are as follows:

```
  1  GATCGACCAG GTGAAGCGGC CGCAGACGGT GGAAGTTCAG CGTCGGGTTT
 51  GTGACGGAGG ACATCCTCCG GGCGCACGTC CCCGAGGGCG GCGACGACAC
101  GCTCGCACTC GCCTGCGGGC CGCCGCCCAT GATCAAGTTC GCCATCTCCC
151  CCAACCTGGA GAAGATGAAG TACGACATGG CCAATTCCTT CATCTCCTTC
201  TGATCGGTGA AACATTTCCC TCTCTAGTAG TACCTACTCA TAGGTAGCAT
251  GGAGCTGCCT AAGCTATATA CACCTACCGT CGACGCATCA ACCTGTGGAT
301  GTGCCCAATG TTTATACGTG TTGTGTTTGT TCATTAGAGA ACCGTACGTA
351  CGTGCATGAA TATGATTGCA TGAATTATAC TGTACGTACA TTGGACATAT
401  ATATACCGAT CGATGTTTTG CCCTCGGGAA GGATGTGAAG TTTACGCCGA
451  TCCGATTGAG GGTGTGTTTG TTCCTTCATG TATACTGCTA CGATAGTATG
501  TAAATAAAGA TTGATTATTC TTCTACCTTA GCCTTGAGAT AAGGGGAATA
551  TTTGAGAATG TATTGGAAAT AGTTTCCAAC AAGGATAATA CAAAGGGAGT
601  TCTTGGTCTG TCTTGAAAAA AAAAAAAAAA AAAAA
```

Sequencing of the bNRp10 clone was carried out, the results for the sequence are as follows: (length 1151 bases, 5' → 3')

```
   1  CTGCAGGGGG GGGGGCCTAC CGCATCGGCG AGCTGATCAC CACGGGGACG
  51  GGGTACAACT CCGACAACTC GGTGCACGGT GGGTCCAGCC TATCCCACCT
 101  CGCACCGATC CGCGAGGCCA CCAAGGTCGC CGGCGCCCCC ATCGCGCTCT
 151  CCAGCCCGCG CGAGAAGGTC CCGTGCCGCC TCGTCGACAA GAAGGAGCTC
 201  TCCCACGACG TCCGCCTCTT CCGTTTCGCG CTGCCGTCCT CCGACCAGGT
 251  GCTCGGCCTG CCCGTTGGCA AGCACATCTT CGTGTGCGCC ACCATCGACG
 301  GCAAGCTCTG CATGCGCGCC TACACGCCGA CGAGCATGGT GGACGAGATC
 351  GGCCAGTTCG AGCTGCTCGT CAAGGTCTAC TTCAGGGACG AGCACCCGAA
 401  GTTCCCCAAC GGCGGGCTCA TGACGCAGTA CCTGGAGTCG CTCCAGGTTG
 451  GCTCTTCCTA CATTGATGTC AAGGGCCCGC TGGGCCATGT GGAGTACACC
 501  GGCCGTGGCA ACTTCGTAAT CAACGGTAAG CAGCGCCGGG CGCGGCGGCT
 551  GGCCATGATC TGCGGCGGCA GCGGGATCAC GCCCATGTAC CAGGTGATCC
 601  AGGCCGTGCT GCGGGACCAG CCGGAGGACG AGACGGAGAT GCACCTGGTG
 651  TACGCCAACC GGAGCGAGGA CGACATCCTG CTGCGCGACG AGCTGGACCG
 701  GTGGGCGGCG GAGTACCCCG ACAGGCTCAA GGTGTGGTAC GTGATCGACC
 751  AGGTGAAGCG GCCGGAGGAC GGGTGGAAGT TCAGCGTCGG GTTCGTGACG
 801  GAGGACATCC TCCGGGCGCA CGTCCCCGAG GGCGGCGACG ACACGCTCGC
 851  ACTCGCCTGC GGGCCGCCGC CCATGATCAA GTTCGCCATC TCCCCCAACC
 901  TGGAGAAGAT GAAGTACGAC ATGGCCAATT CCTTCATCTC CTTCTGATCG
 951  GTGAAACATT TCTCCTCTCT AGTAGTACCT ACTCATAGGT AGCATGGAGC
1001  TGCCTAAGCT ATATACACCT ACGCGTCGAC GCATCAACCT GTGGATGTGC
1051  CCAATGTTTA TACGTGTTGT GTTTGTTCAT TAGAGAACCG TACGTACGTG
1101  CATGAATATG ATTGCATGAA AAAAAAAAAA AAAACCCCCC CCCCCCTGCA
1151  G
```

EXAMPLE 6

Cloning the Nitrate Reductase Gene from Arabidopsis

The nitrate reductase (NR) gene from the dicotyledous Arabidopsis thaliana was also cloned.

The mRNA of arabidopsis was enriched by sucrose gradiant size fractionation prior to cDNA synthesis, as described in Example 2. The fractions containing the nitrate reductase mRNA were identified by in vitro translation and immunoprecipitation with barley nitrate reductase antibody. This population of mRNA, enriched for NR mRNA, was used to construct a cDNA library in an expression vector. Double stranded cDNAs were synthesized according to Gubler and Hoffman, Gene, 25:263 (1983) and, after EcoRI linker addition, were cloned into λgt11. The recombinant plaques were screened with NR antibody. Four cDNA clones were identified, ranging in size from 2.3-2.5 Kb.

Although the foregoing invention has been described in some detail by way of illustration and example for purposes of clarity of understanding, it will be obvious that certain changes and modifications may be practiced within the scope of the invention, as limited only by the scope of the apended claims.

## Claims

1. A recombinant polynucleotide molecule containing genetic information coding for nitrate reductase.

2. The molecule of claim 1 which is a replication vehicle.

3. The molecule of claim 1 wherein said polynucleotide is or is derived from cDNA.

4. A replication vehicle comprising a polynucleotide fragment containing genetic information coding for nitrate reductase and conferring nitrate reductase phenotype upon a nitrate reductase deficient host.

5. A method for selecting transformed host cells comprising transforming a nitrate reductase deficient host with a polynucleotide fragment containing genetic information coding for nitrate reductase.

6. A method for producing a heterologous polypeptide in a host transformed to express said heterologous polypeptide comprising:

(a) transforming a nitrate reductase deficient host cell with a polynucleotide fragment containing genetic information coding for nitrate reductase and with a heterologous polypeptide expressible in said host;

(b) selecting host cells transformed by said nitrate reductase;

(c) culturing said transformed host in a culture medium under culturing conditions; and

(d) recovering said polypeptide from said culture.

Neu eingereicht / Newly ...ed
Nouvellement dépos...

NITRATE REDUCTASE
IS INDUCED BY NITRATE

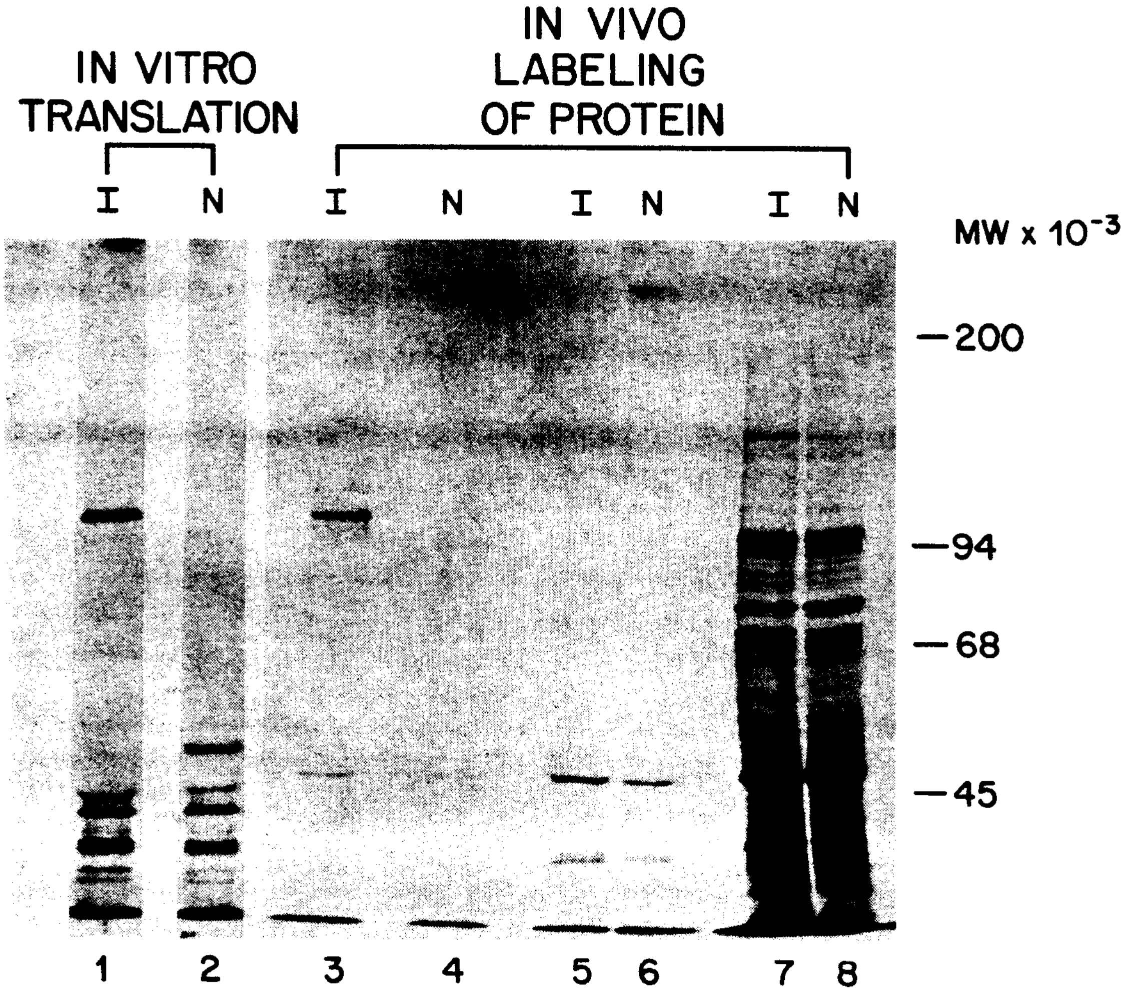


FIG. 1

FRACTIONATION OF mRNA ON SUCROSE GRADIENT,

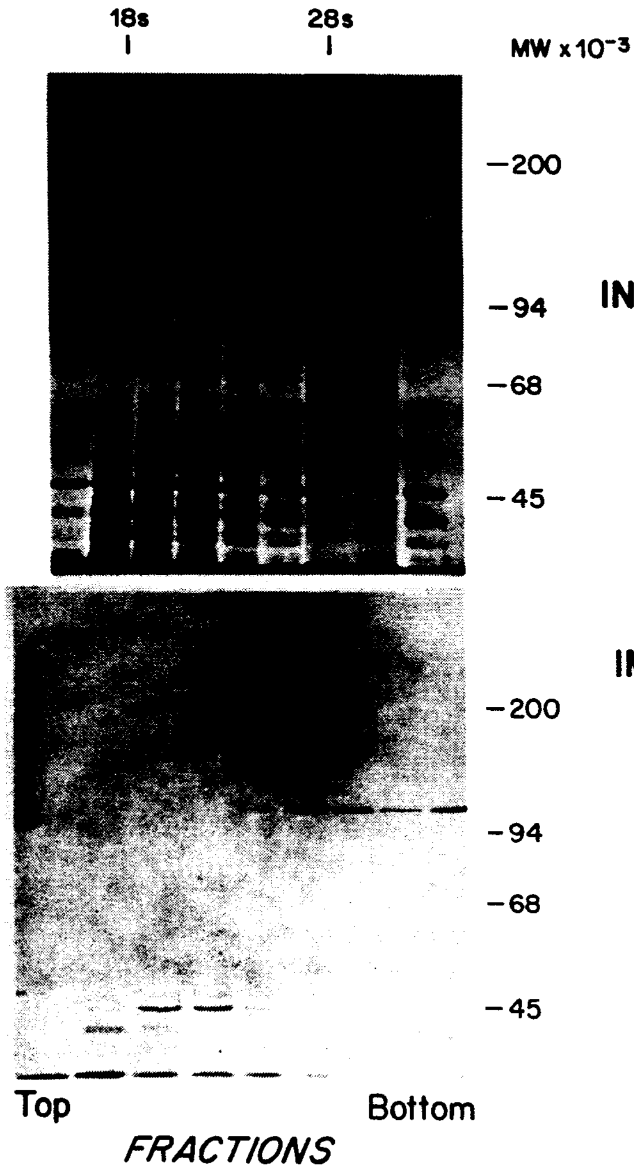


FOLLOWED BY

IN VITRO TRANSLATION

AND

IMMUNOPRECIPITATION

FIG. 2

FRACTIONATION OF mRNA IN METHYLMERCURY HYDROXIDE ACAROSE GEL,

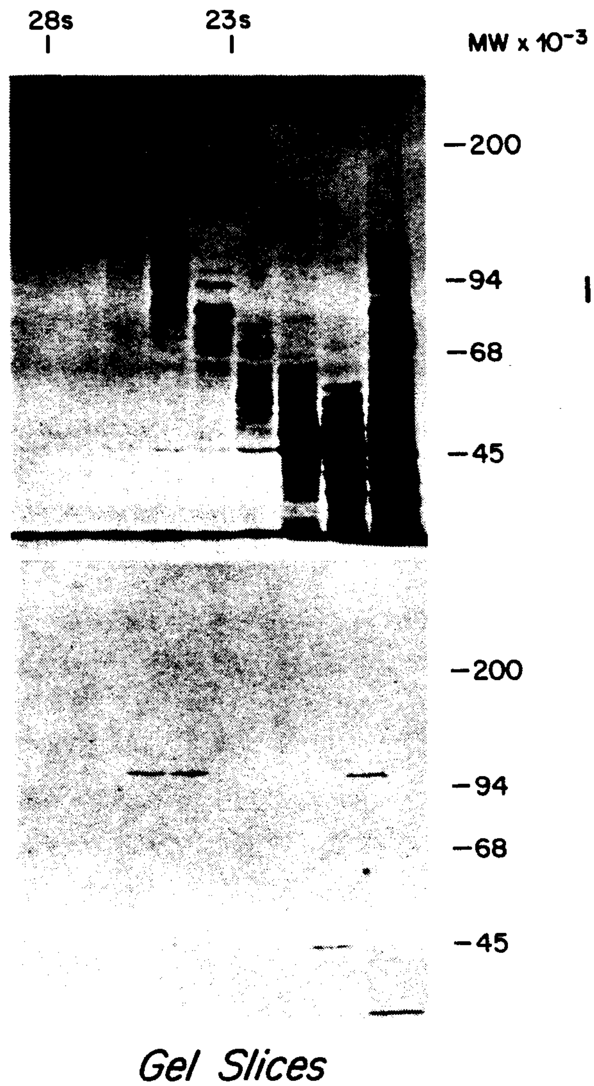


FOLLOWED BY

IN VITRO TRANSLATION

AND

IMMUNOPRECIPITATION

FIG. 3

# NORTHERN ANALYSIS

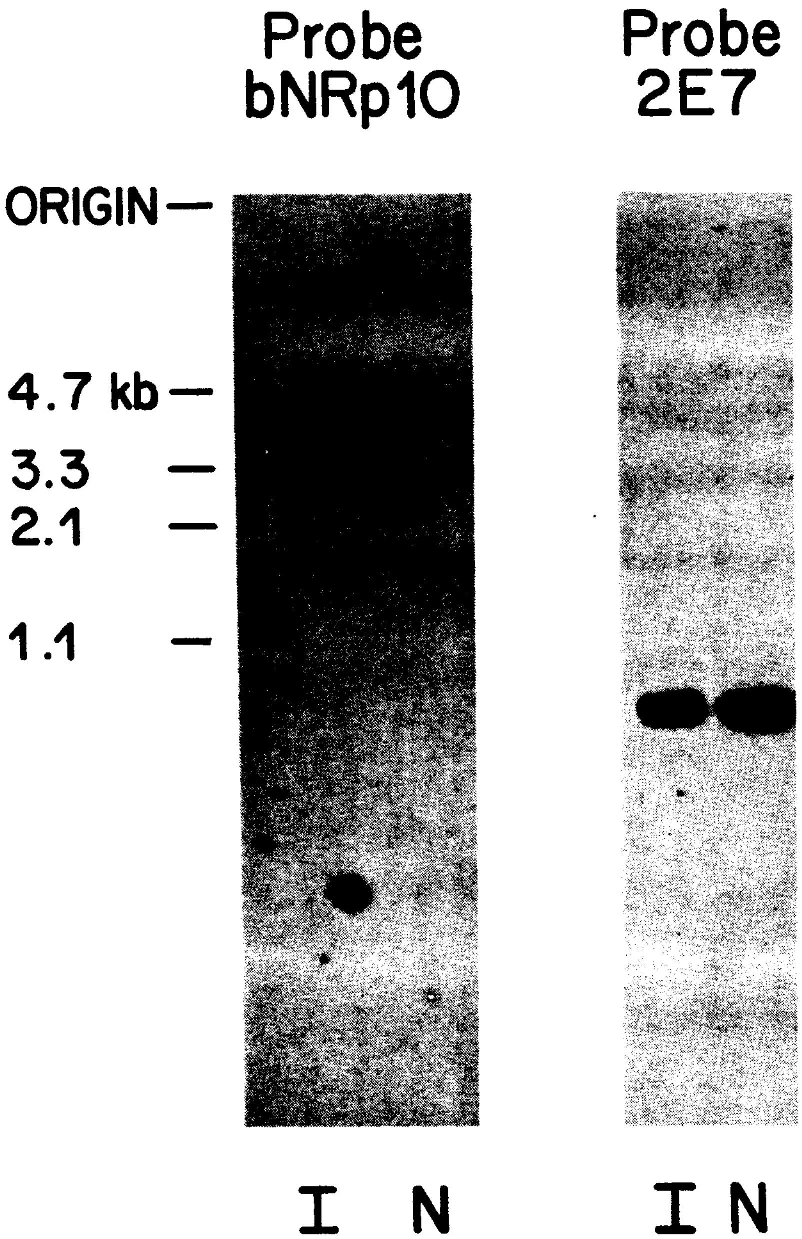


FIG. 4

HYBRID SELECTION WITH cDNA bNRp10
FOLLOWED BY IN VITRO TRANSLATION
AND IMMUNO PRECIPITATION

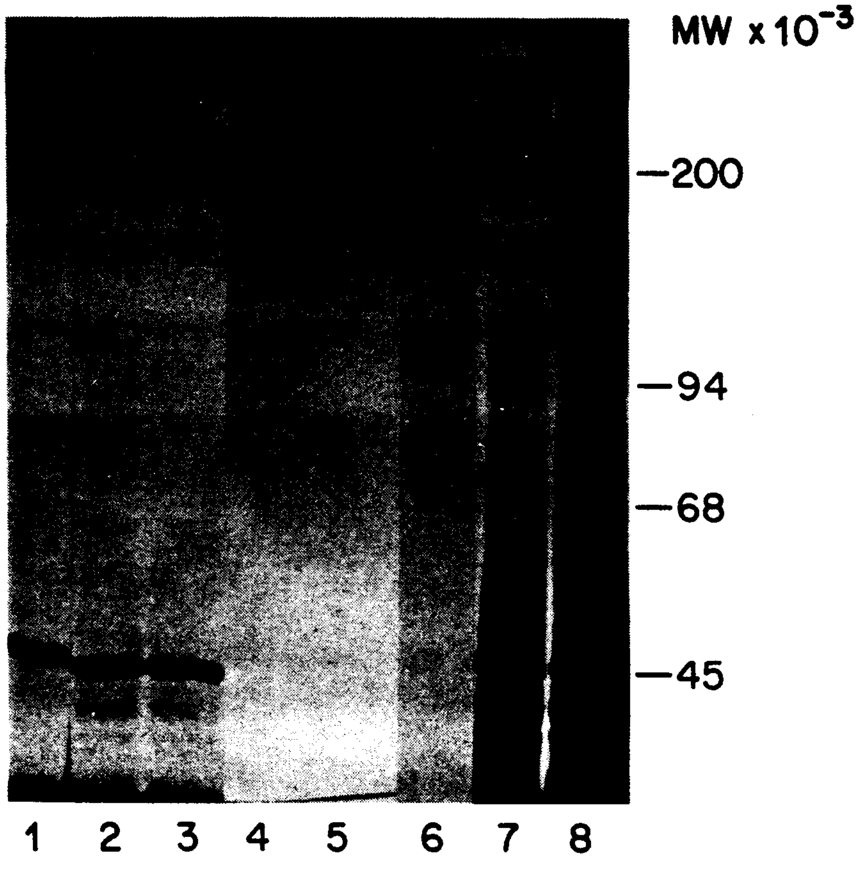


FIG. 5

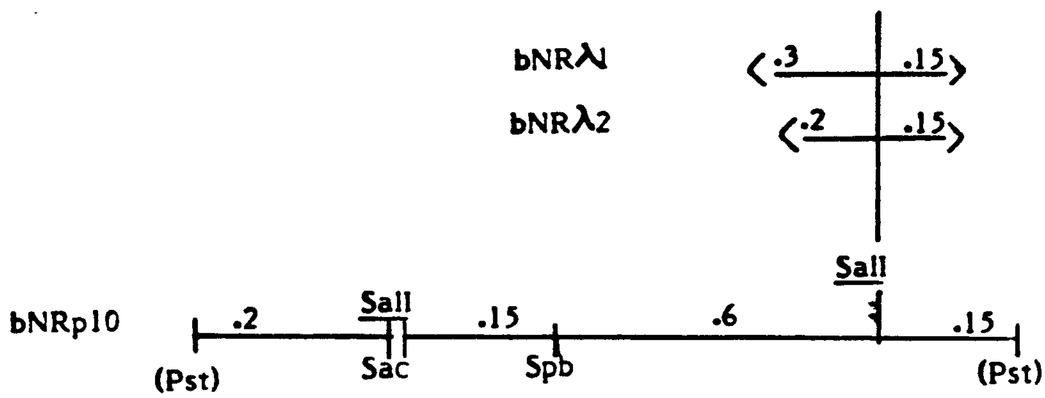


* sizes are approximate, in Kb

** (Pst) - sites created at the ends to place into vector

FIG. 6

European Patent Office

# EUROPEAN SEARCH REPORT

Application number

EP 86 11 4978

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| X,P | PROCEEDINGS OF THE NATIONAL ACADEMY OF SCIENCES USA, vol. 83, September 1986, pages 6825-6828; C.-L. CHENG et al.: "Cloning and nitrate induction of nitrate reductase mRNA" * Whole document * | 1-4 | C 12 N 15/00 |
| Y | Idem | 5,6 | |
| X | CHEMICAL ABSTRACTS, vol. 99, no. 9, 29th August 1983, page 174, abstract no. 65314u, Columbus, Ohio, US; T, MANZARA: "Construction of recombinant Ti plasmids containing the chlM gene from E. coli", & NATO ADV. SCI. INST. SER., SER. A 1983, 61(Genet. Eng. Eukaryotes), 125-31 * Abstract * | 1,2 | TECHNICAL FIELDS SEARCHED (Int. Cl.4) C 12 N |
| Y | Idem | 4-6 | |
| | -/- | | |

The present search report has been drawn up for all claims

| Place of search | Date of completion of the search | Examiner |
|---|---|---|
| THE HAGUE | 09-02-1987 | YEATS S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

EP 86 11 4978

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 99, no. 9, 29th August 1983, page 174, abstract no. 65315v, Columbus, Ohio, US; A. KLEINHOFS et al.: "Nitrate reductase genes as selectable markers for plant cell transformation", & NATO ADV. SCI. INST. SER., SER. A 1983, 61(Genet. Eng. Eukaryotes), 215-31 * Abstract * | 1,2 | |
| Y | Idem | 4-6 | |
| X | CHEMICAL ABSTRACTS, vol. 100, no. 9, 27th February 1984, page 137, abstract no. 62678u, Columbus, Ohio, US; J.L. TAYLOR et al.: "Reconstitution of plant nitrate reductase by Escherichia coli extracts and the molecular cloning of the chlA gene of Escherichia coli K12", & J. MOL. APPL. GENET. 1983, 2(3), 261/71 * Abstract * | 1,2 | TECHNICAL FIELDS SEARCHED (Int. Cl.4) |
| Y | Idem | 4-6 | |
| | -/- | | |

The present search report has been drawn up for all claims

| Place of search | Date of completion of the search | Examiner |
|---|---|---|
| THE HAGUE | 09-02-1987 | YEATS S.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application number

EP 86 11 4978

## DOCUMENTS CONSIDERED TO BE RELEVANT



| Category | Citation of document with indication, where appropriate, of relevant passages | Relevant to claim | CLASSIFICATION OF THE APPLICATION (Int. Cl.4) |
|---|---|---|---|
| A | JOURNAL OF CELLULAR BIOCHEMISTRY SUPPLEMENT US, vol. 0, no. 9, part C, 1985, page 232; C.L. CHENG et al.: "The cloning of the plant nitrate reductase gene(s)" * Abstract * | 1-3 | |
| | ----- | | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 09-02-1987 | YEATS S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82